Europäisches Patentamt

European Patent Office

Office européen des brevets

(19)

(11) Numéro de publication: **0 089 280 B1**

(12) **FASCICULE DE BREVET EUROPEEN**

(45) Date de publication de fascicule du brevet:
24.07.91

(51) Int. Cl.⁵: **A61K 9/20, A61K 9/48**

(21) Numéro de dépôt: 83400491.3

(22) Date de dépôt: 10.03.83

(54) **Comprimé en forme de gelule, procédé et dispositif pour sa préparation.**

(30) Priorité: 15.03.82 CH 1594/82

(43) Date de publication de la demande:
21.09.83 Bulletin 83/38

(45) Mention de la délivrance du brevet:
24.07.91 Bulletin 91/30

(84) Etats contractants désignés:
AT BE CH DE GB IT LI LU NL SE

(56) Documents cités:
DE-A- 2 834 226
FR-A- 1 107 548
FR-A- 1 153 998
GB-A- 1 561 100

(73) Titulaire: MICROENCAPSULATION S.A. Société dite:
35 Corso San Gottardo
Chiasso Tessin(CH)

(72) Inventeur: Scapinelli, Bruno
5 Allée Guynemer Domaine de la Maisonnière
F-60260 Lamorlaye(FR)

(74) Mandataire: Ores, Irène et al
CABINET ORES 6, Avenue de Messine
F-75008 Paris(FR)

EP 0 089 280 B1

## Description

La présente invention est relative à à un procédé et à un dispositif pour réaliser la préparation de comprimés en forme de gélule.

Comme il est bien connu, de nombreuses compositions pharmaceutiques spécialement dans les cas où il est nécessaire de masquer la saveur et/ou l'odeur, ou dans les cas où la préparation pharmaceutique doit arriver dans l'estomac ou dans les intestins sans avoir été altérée, comme pour les produits pharmaceutiques à action prolongée ou retardée, sont préparées, pour l'administration, en forme de gélules constituées par une enveloppe extérieure qui peut être dure ou molle, généralement en gélatine et en substances neutres similaires, et contenant les granules de matière première.

Toutefois, de telles gélules sont de dimensions relativement grandes et de nombreuses personnes ont des difficultés à les avaler ; la matière neutre de l'enveloppe qui se dissout dans l'estomac ou l'intestin, cause fréquemment des troubles et en outre, leur préparation et leur production requiert toute une série d'opérations et d'appareillages qui la rendent relativement coûteuse par rapport aux autres formes plus simples d'administration traditionnelle.

D'un autre côté, il serait impossible de préparer un comprimé normal contenant des granules ou des microgranules de substances actives et surtout des granules à action prolongée ou retardée qui ne doivent pas être endommagés pour ne pas modifier le temps et le mode de libération du principe actif, vu que les machines à comprimer, qui exercent une forte compression sur un disque large et de faible hauteur, détruiraient l'intégrité d'une grande partie des granules ou des microgranules.

La présente invention résout ce problème, très important dans le domaine pharmaceutique, et fournit un procédé pour l'obtention d'un comprimé qui présente la forme d'une gélule et qui est constitué par un excipient neutre qui englobe les granules ou les microgranules de matière première en gardant parfaitement l'intégrité de ceux-ci sans altérer les temps et les modes de libération des principes actifs qu'ils contiennent.

Naturellement, aussi bien les excipients (tels que l'amidon par exemple) que les matières premières devront être aptes au but recherché. Le comprimé obtenu selon la présente invention pourra avantageusement remplacer les gélules de gélatine traditionnelles avec les avantages par rapport à celles-ci de pouvoir être ingérées plus facilement et surtout de libérer immédiatement les granules dans l'estomac ou dans les intestins si ce comprimé est fabriqué avec des excipients gasto-résistants.

Le procédé selon l'invention est essentiellement caractérisé par le fait que le mélange d'un excipient neutre englobant les granules de matière première est comprimé dans le sens de la longueur (plutôt que dans le sens de la largeur comme dans les machines à comprimer usuelles), c'est-à-dire en exerçant la pression sur les deux têtes du comprimé et donc sur des petites surfaces par rapport à la masse du comprimé, ce qui fait que les granules englobés dans ladite masse ne subissent aucune sollicitation propre à altérer leurs caractéristiques physico-chimiques.

Un autre avantage important du comprimé en forme de gélule obtenu selon la présente invention, avantage qui est seulement facultatif et non pas limitatif, consiste dans le fait que ce comprimé est divisible en fractions et que donc l'administration du médicament peut être fractionnée en plusieurs doses, chose qui est évidemment impossible pour les gélules.

De préférence, mais non pas forcément, la forme du comprimé objet de la présente invention, reproduit exactement celle des gélules de gélatine qui, comme il est connu, sont composées de deux demi-capsules emboîtées l'une dans l'autre, en sorte que le point de séparation du comprimé en deux morceaux est représenté par le gradin qui symbolise le point de jonction des deux demi-gélules, la quantité de substance active contenue dans chacune des deux moitiés du comprimé sera égale et on peut donc être sûr d'administrer des demi-doses égales entre elles.

Le dispositif pour mettre le procédé en oeuvre et préparer, dans la pratique, les comprimés en forme de gélule selon la présente invention, est caractérisé par le fait qu'il se compose d'une matrice qui contient au moins une chambre verticale dans laquelle on introduit le mélange d'un excipient approprié et des granules de matière active, qui est comprimé entre un poinçon supérieur et un poinçon inférieur dont chacun présente à son extrémité ou tête qui agit sur le mélange, la forme en ogive de la tête ou ogive correspondante du comprimé en forme de gélule que l'on veut obtenir. Naturellement pour la production à l'échelle industrielle, la matrice comprend une série de chambres pour la formation de comprimés avec autant de poinçons supérieurs et inférieurs qui seront actionnés en synchronisme et le dispositif est doté de tous les mécanismes et automatismes normaux des machines pour la production d'articles à grande échelle.

Outre les dispositions qui précèdent, l'invention comprend encore d'autres dispositions, qui ressortiront de la description qui va suivre.

L'invention sera mieux comprise à l'aide du complément de description qui va suivre, qui se réfère au dessin annexé dans lequel :

- la figure 1 est une vue latérale du comprimé en forme de gélule selon la présente invention ;

- la figure 2 est une vue schématique en coupe du dispositif pour la production du comprimé de la figure 1, et
- la figure 3 est une vue schématique en coupe du dispositif de la figure 2 en position de formation d'un comprimé avec les poinçons fermés.

Il doit être bien entendu, toutefois, que ce dessin et les parties descriptives correspondantes, sont donnés uniquement à titre d'illustration de l'objet de l'invention, dont ils ne constituent en aucune manière une limitation.

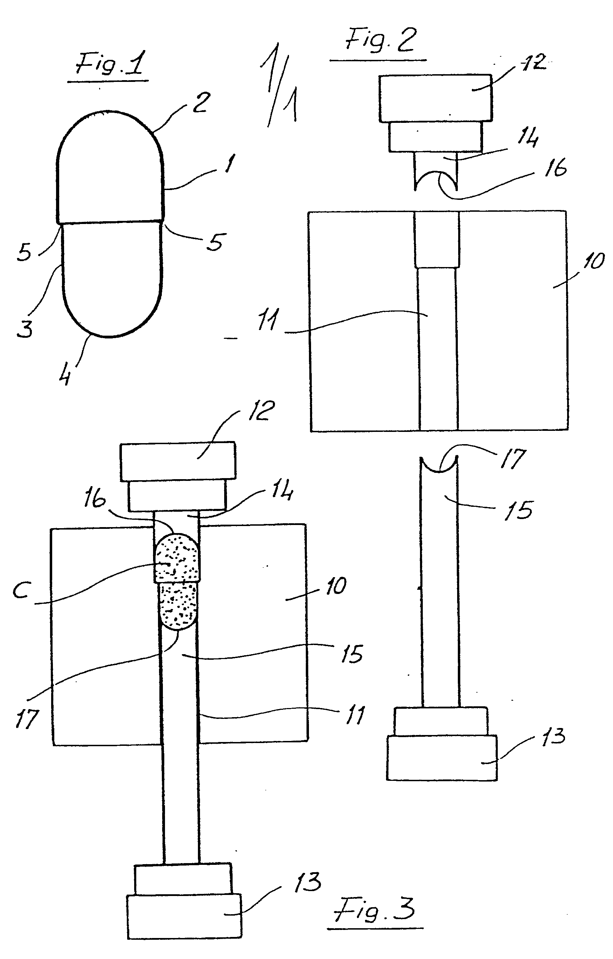

En faisant référence aux figures du dessin annexé, dans la figure 1, on voit un comprimé en forme de gélule selon la présente invention qui reproduit exactement la forme des gélules de gélatine et comprend la demi-gélule supérieure 1, avec sa tête semi-sphérique ou ogive 2, et la demi-gélule inférieure 3 avec sa tête semi-sphérique ou ogive 4 délimitée par la ligne ou gradin de séparation 5 le long de laquelle le comprimé peut être éventuellement divisé en deux.

Comme déjà mentionné ci-dessus, on doit toutefois comprendre que le comprimé pourrait aussi ne pas comporter le gradin 5 ou comporter plus d'un gradin pour pouvoir le subdiviser en un nombre supérieur de fractions.

En examinant maintenant les figures 2 et 3 du dessin annexé, le dispositif pour la production des comprimés est constitué par une matrice 10 qui contient au moins une chambre verticale 11, mais de préférence une série de chambres disposées en rangées, dans laquelle on introduit le mélange d'un excipient approprié (par exemple de l'amidon) et des granules de substance active, qui est comprimé entre un poinçon supérieur 12 et un poinçon inférieur 13 dont chacun présente à son extrémité ou tête 14 ou 15 qui agit sur le mélange, la forme ou empreinte 16 ou 17 de la tête semi-sphérique ou ogive 2 ou 4 correspondante, du comprimé en forme de gélule (C) qui est ainsi formée par compression entre lesdits poinçons, exercée dans le sens de la longueur du comprimé sur ses deux têtes ou ogives 2 et 4, sans pour autant endommager les granules englobés dans le mélange.

## Revendications

1. Procédé d'obtention de comprimés présentant la forme de gélules qui se composent de deux demi-capsules (1,3) avec les têtes ou ogives (2,4) correspondantes, qui sont constituées par un mélange d'excipients neutres, englobant la substance active sous forme de granules ou de microgranules, lesdites gélules pouvant être divisibles en fractions déterminées par des lignes le long desquelles le comprimé peut être subdivisé, lequel procédé est caractérisé par le fait que le mélange d'excipients neutres qui englobe les granules de matière première est comprimé dans le sens de la longueur, c'est-à-dire en exerçant une pression sur les deux ogives du comprimé et donc sur des petites surfaces par rapport à la masse du comprimé, de telle manière que l'intégrité des granules englobés dans ladite masse n'est pas endommagée.

2. Procédé selon la revendication 1, caractérisé par le fait que l'on forme sur les comprimés un ou plusieurs gradins ou sillons pour permettre la subdivision du comprimé en fractions.

3. Procédé selon la revendication 2, caractérisé par le fait que le gradin est formé en correspondance du point de jonction des deux fractions en forme de demi-gélules du comprimé.

4. Dispositif pour la production de comprimés présentant la forme de gélules selon la revendication 1 en mettant en oeuvre le procédé selon l'une quelconque des revendications 1 à 3, caractérisé par le fait qu'il est constitué par une matrice (10) qui contient au moins une chambre verticale (11) dans laquelle on introduit le mélange d'excipients convenables et de granules de substance active, et par un poinçon supérieur (12) et un poinçon inférieur (13) dont chacun présente à son extrémité ou tête (14,15) qui agit sur le mélange, la forme ou l'empreinte (16,17) de la tête ou ogive correspondante (2,4) du comprimé en forme de gélule que l'on veut produire, et par des moyens pour mouvoir lesdits poinçons à l'intérieur de la chambre de la matrice, de manière à comprimer ledit mélange en correspondance des deux têtes ou ogives, et produire de cette façon le comprimé.

5. Dispositif selon la revendication 4, caractérisé par le fait que la chambre de la matrice présente un ou plusieurs gradins aptes à provoquer sur le comprimé qui est formé à l'intérieur de ladite chambre, les gradins ou lignes de subdivision en fractions de comprimé.

## Claims

1. Method of producing capsule-shape tablets which are composed of two half-capsules (1,3) with corresponding caps or shells (2,4), which are constituted by a mixture of neutral excipients coating the active substance in granule or microgranule form, and said capsules can be divisible into particular fractions bounded by lines along which the tablet can be subdivised, said method being characterized by the fact that the neutral excipient mixture which coats the granules of raw material is compressed in the longitudinal direction, that is to say that by exerting a pressure on the two caps of the tablet and hence on the small surfaces with respect to the mass of the tablet, so that the integrity of the coated granules in said mass is undamaged.

2. Method according to claim 1, wherein one or several steps or sills are formed on the tablets to enable subdivision of the tablet into fractions.

3. Method according to claim 2,wherein the step is formed in correspondence with the junction point of the two fractions in the form of half-gelules of the tablet.

4. Device for the production of tablets having gelule form according to claim 1, by employing the method according to any one of claims 1 to 3, characterized by the fact that it is constituted by a matrix (10) which contains at least one vertical chamber (11) into which the mixture of suitable excipients and of granules of active substance are introduced and by an upper punch (12) and a hover punch (13) of which each has at its end or head (14,15) which acts on the mixture, the shape or impression (16,17) of the head or corresponding shell (2,4) of the capsule-shape tablet which it is desired to produce, and by means to move said punches within the chamber of the matrix, so as to compress said mixture in correspondence with the two heads or shells, and thus to produce said tablet.

5. Device according to claim 4, wherein the chamber of the matrix has one or several steps adapted to cause on the tablet which is formed within said chamber, the steps or lines of tablet subdivision intro fractions.

**Patentansprüche**

1. Verfahren zur Herstellung von Tabletten in Form von Kapseln, die aus zwei Halbkapseln (1, 3) mit einander entsprechenden Köpfen oder Spitzen (2, 4) und die aus einer Mischung neutraler Grundmassen bestehen, welche die in Form eines Granulats oder Mikrogranulats vorliegende aktive Substanz umschließt, wobei die Kapseln mittels Linien, längs derer die Tablette unterteilt werden kann, in vorbestimmte Bruchteile teilbar sind und das Verfahren dadurch gekennzeichnet ist, daß die Mischung neutraler Grundmassen, welche die Granualien des Ausgangsstoffes umschließen in Längsrichtung komprimiert wird, nämlich durch Ausüben eines Druckes auf die beiden Spitzen der Tablette und folglich auf bezüglich der Masse der Tablette kleine Oberflächen in einer solchen Weise, daß die Unversehrtheit der in der genannten Masse eingeschlossenen Granualien nicht beeinträchtigt wird.

2. Verfahren nach Anspruch 1, dadurch gekennzeichnet, daß auf den Tabletten ein oder mehrere Stufen oder Rillen ausgeformt werden, um die Unterteilung der Tablette in Bruchteile zu ermöglichen.

3. Verfahren nach Anspruch 2, dadurch gekennzeichnet, daß die Stufe in Übereinstimmung mit der Verbindungsstelle der zwei Tablettenabschnitte in Form von Halbkapseln geformt ist.

4. Vorrichtung zur Herstellung von Tabletten in Form von Kapseln nach Anspruch 1 unter Verwendung des Verfahrens nach irgendeinem der Ansprüche 1 bis 3, dadurch gekennzeichnet, daß sie aus einer Matrize (10) besteht, welche zumindest eine vertikale Kammer (11) aufweist, in welche die Mischung geeigneter Grundstoffe und Granualien der aktiven Substanz eingebracht werden, sowie aus einem oberen Stempel (12) und aus einem unteren Stempel (13), deren jeder an seinem auf die Mischung wirkenden Ende oder Kopf (14, 15) die Form oder den Abdruck (16, 17) des entsprechenden Kopfes bzw. der Spitze (2, 4) der herzustellenden Tablette in Form einer Kapsel aufweist, und daß Mittel vorgesehen sind, um die Stempel so in das Innere der Matrizenkammer zu bewegen, daß die Mischung in Übereinstimmung mit den beiden Köpfen oder Spitzen komprimiert und solcherweise die Tablette hergestellt wird.

5. Verfahren nach Anspruch 4, dadurch gekennzeichnet, daß die Matrizenkammer ein oder mehrere Stufen aufweist, die geeignet sind, um auf der im Inneren der genannten Kammer geformten Tablette die Stufen oder Linien zur Unterteilung der Tablette in Bruckstücke hervorzurufen.

Fig.1

Fig.2

Fig.3